# EUROPEAN PATENT SPECIFICATION

(11) **EP 4 448 169 B1**
(45) Date of publication and mention of the grant of the patent: **13.08.2025**
(21) Application number: 22826400.8
(22) Date of filing: 13.12.2022
(51) Int. Cl.: B01J 31/16, B01J 21/18, C07C 17/08, B01J 37/02, B01J 31/22, B01J 27/02, B01J 23/52, B01J 35/40

(54) **GOLD CONTAINING CATALYST, METHOD OF PREPARATION AND USE**
GOLDHALTIGER KATALYSATOR, VERFAHREN ZUR HERSTELLUNG UND VERWENDUNG
CATALYSEUR CONTENANT DE L'OR, PROCÉDÉ DE PRÉPARATION ET UTILISATION

(30) Priority: 17.12.2021 EP 21386079
(43) Date of publication of application: 23.10.2024
(73) Proprietor: Johnson Matthey Public Limited Company, London EC2V 7AD (GB)
(72) Inventor: CARTHEY, Nicholas, Sonning Common RG4 9NH (GB); DUMMER, Nicholas Francois, Cardiff CF24 0DE (GB); HUTCHINGS, Graham John, Cardiff CF24 0DE (GB); JOHNSTON, Peter, Royston, SG8 5HE (GB); LAZARIDOU, Anna, Cardiff CF24 0DE (GB); PATTISSON, Samuel David, Cardiff CF24 0DE (GB); SMITH, Louise Rhiannon, Cardiff CF24 0DE (GB); SMIT, Joost, London W2 6LG (GB)
(74) Representative: Johnson Matthey Plc
(86) International application number: PCT/GB2022/053185
(87) International publication number: WO 2023/111537

(56) References cited:
- WO-A1-2020/016555
- WO-A1-2020/254817
- CN-A- 109 847 802
- MÖHRLE HANS ET AL: "Doppelsalzbildung bei Amiden und vinylogen Amiden", MONATSHEFTE FÜR CHEMIE, 1 January 1978 (1978-01-01), pages 1295 - 1303, XP055926621, Retrieved from the Internet <URL:https://link.springer.com/content/pdf/10.1007/BF00906041.pdf> [retrieved on 20220531]
- LIU YANXIA ET AL: "Progress and Challenges of Mercury-Free Catalysis for Acetylene Hydrochlorination", CATALYSTS, vol. 10, no. 10, 20 October 2020 (2020-10-20), CH, pages 1218, XP055926275, ISSN: 2073-4344, DOI: 10.3390/catal10101218

## Description

### Field of the Invention

The present invention relates to gold containing catalysts, particularly for the conversion of acetylene to vinyl chloride monomer (VCM).

### Background

The hydrochlorination of acetylene to produce VCM as the precursor to polyvinyl chloride (PVC) is currently a large scale industrial process, particularly in coal rich areas such as China and in areas rich in natural gas through natural gas to acetylene routes. Over 20 million tonnes of VCM are produced annually through acetylene hydrochlorination with the vast majority utilising mercuric chloride (HgCl₂) catalysts supported on activated carbon. The mercury catalyst poses significant environmental concerns due to volatile HgCl₂ subliming from the catalyst bed, up to 0.6 kg Hg/tonne VCM production. Due to the environmental impact of this process, the recently ratified Minamata convention dictates that all new VCM plants must use mercury free catalysts and in the near future all existing industrial plants must switch to mercury free alternatives. This has revived the commercial interest in using gold and other metals as a catalyst for this reaction.

It is known from WO2010/055341 (Johnson Matthey PLC and Aker Process B.V.) that Au-containing catalysts prepared by wet impregnation of a mixture of HAuCl₄ / aqua regia are active for the conversion of acetylene to VCM. The catalysts are believed to include gold particles having a metallic gold core and a shell of higher oxidation state species, including Au(I) and Au(III) which are believed to be the active species for the hydrochlorination reaction.

WO2013/008004 (Johnson Matthey PLC) describes a development of catalysts based on HAuCl₄ by including a sulphur-containing ligand such as thiosulphate. It is thought that the sulphur-containing ligand forms a complex with the Au atoms thereby stabilizing Au(I) and Au(lll) which are the active species in the hydrochlorination reaction.

A further development of the above catalysts is described in WO2020/254817 (Johnson Matthey PLC), in which an inorganic oxide, hydroxide, oxo-salt or oxo-acid is included in order to improve the resistance of the catalyst towards carbon nanotube formation.

VCM catalysts comprising a complex of gold with a thiosulphate ligand on a carbon support have been commercialised by Johnson Matthey under the brand PRICAT^{™} MFC. While these catalysts have been a commercial success, the very high initial activity of these catalysts in the extremely exothermic acetylene hydrochlorination reaction needs to be carefully optimised for a specific system. If the catalyst activity is too high initially then local hot spots may be formed in the catalyst bed which can cause deactivation. There is a need for alternative catalysts which have a more stable activity profile and which are less prone to initial overheating. The present invention addresses this problem.

### Summary of Invention

The present inventors have found that catalysts with stable activity profiles which are less prone to initial overheating can be prepared by modifying the production method described in WO2013/008004 and WO2020/254817. The methods exemplified in these references involve the formation of an aqueous impregnation solution containing a complex of gold with a sulphur-containing ligand which is applied onto a carbon support. The inventors have surprisingly found that the initial activity of the catalyst can be reduced by replacing the aqueous solvent of the impregnation solution with an organic solvent or a mixture of organic solvent and aqueous solvent. Without wishing to be bound by theory, this change is thought to alter wetting between the catalyst and the impregnation solution, which alters the dispersion of gold complex on the support.

In a first aspect the invention relates to a method of manufacturing a hydrochlorination catalyst, comprising the steps of:
i) preparing an impregnation solution by combining a source of gold and a ligand in a solvent, wherein the solvent comprises an organic solvent which comprises or consists of acetone;
ii) impregnating a support with the impregnation solution from step (i); and
iii) drying the product of step (ii) to obtain the catalyst;
wherein the ligand is of Formula (I) 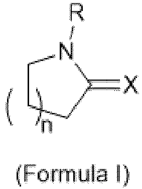 wherein
X is O or S;
n is 1 or 2;
R is H or a C1 to C10 hydrocarbon group optionally including one or more heteroatoms selected from halogen (F, Cl, Br, I), oxygen or nitrogen.

WO2020/016555 (University College Cardiff Consultants Ltd) describes a method of producing hydrochlorination catalysts by combining gold, an organic solvent and a support material. In the examples of this reference catalysts are prepared by impregnating a solution of HAuCl₄·3H₂O in an organic solvent. An advantage of this method is that no ligand is required. The method according to the first aspect is similar to the method in WO2020/016555, but requires that impregnation is carried out using an impregnation solution containing a ligand in addition to the source of gold.

In a second aspect the invention relates to a catalyst comprising a complex of gold and a ligand of Formula (I) 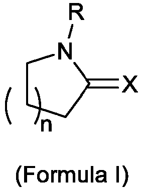 wherein
X is O or S;
n is 1 or 2;
R is H or a C1 to C10 hydrocarbon group optionally including one or more heteroatoms selected from halogen (F, Cl, Br, I), oxygen or nitrogen; and
the complex is supported on a support.

It is not yet fully understood whether catalysts comprising gold and a ligand of Formula I are coordination complexes (e.g. of formula Au[ligand]₄₋ₓClₓ where x = 1 to 4) or adducts (e.g. [AuCl₃]·ligand). It is possible that this varies depending on the choice of ligand. As used herein, the term "complex" covers both possibilities. For the avoidance of doubt, the oxidation state of Au within the complex may be +1 or +3.

It is known that complexes of copper and nitrogen-containing ligands can be used as catalysts in the conversion of acetylene to VCM. CN111774094A and CN111715253A (Nankai University) describe a method for producing a copper-based catalyst for the production of vinyl chloride. The method involves a step of impregnating a carbon support with an impregnation solution containing copper chloride and various additives, which may be nitrogen-containing ligands. CN110743624A (Zhejiang University of Technology) describes a comparative example, prepared by combining a solution of CuCl₂.2H₂O and N-methyl pyrrolidone (NMP) with activated carbon followed by drying and treatment with hydrogen chloride gas. To the inventors' best knowledge, analogous complexes of gold and NMP have not previously been described.

In a third aspect the invention relates to a process for the catalytic hydrochlorination of a substrate containing an alkyne unit, wherein the reaction is carried out in the presence of a catalyst according to the second aspect, or a catalyst produced by or producible by a method according to the first aspect.

The process is particularly suitable when acetylene is used as the substrate, to produce VCM. The conversion of acetylene to VCM is preferably carried out in the gas phase.

### Description of the Figures

Figure 1 shows the activity of catalysts E1 to E11 over time.
Figure 2 shows the activity of catalysts E2-1.2, E2-2.0 and E2-4.0 over time.
Figure 3 shows the NMR spectrum of NMP and the complex formed by combining HAuCl₄·3H₂O and NMP in a 1 : 4 molar ratio.
Figure 4 shows the activity of catalyst E2-1.2 at varying temperatures.
Figure 5 shows the activity of catalysts E12 to E18 over time.
Figure 6 shows the activity of catalysts E19 (comparative) and E20 over time.
Figure 7 shows the activity of catalysts E21 and E22 over time.
Figure 8 shows the single crystal X-Ray structure of [HAuCl₄](NMP)₂.

### Detailed Description

Herein all % relate to percentages by weight of the total catalyst, unless otherwise stated.

### Manufacture of the catalyst

According to a first aspect of the invention, there is provided a method of manufacturing a hydrochlorination catalyst comprising the steps of:
i) preparing an impregnation solution by combining a source of gold and a ligand of Formula (I) in a solvent, wherein the solvent comprises an organic solvent which comprises or consists of acetone;
ii) impregnating a support with the impregnation solution from step (i); and
iii) drying the product of step (ii) to obtain the catalyst.

It is thought that in step (i) a complex is formed between the gold and the ligand. It is preferred that the impregnation solution is prepared by adding the ligand to a solution containing the source of gold and the solvent. However, it is also possible to add the source of gold to a solution containing the ligand and the solvent. It is also expected that the catalyst could be prepared by treating the support sequentially with the source of gold followed by the ligand, or vice versa.

The source of gold is typically a gold salt which is able to form a complex with the ligand. A preferred source of gold is HAuCl₄, either as a solid (e.g. HAuCl₄·3H₂O) or as a solution (e.g. HAuCl₄ in HCl/water)

The solvent comprises an organic solvent which comprises or consists of acetone. The presence of an organic solvent in the impregnation solution is thought to alter wetting between the impregnation solution and the support, leading to improved dispersion of gold in comparison to when an aqueous solution of gold complex is used as the impregnation solution.

In some embodiments the solvent consists of acetone. This may be preferable where both the source of gold and the ligand are soluble in acetone.

In some embodiments the solvent comprises a mixture of an organic solvent and an aqueous solvent. This may be necessary where one of the source of gold or ligand are poorly soluble in the organic solvent. For example, where the source of gold is soluble in organic solvent but the ligand is poorly soluble in organic solvent, the ligand may be dissolved in aqueous solvent and then added to the solution of gold in organic solvent.

Suitable organic solvents are described in WO2020/016555, the contents of which are incorporated herein by reference.

In some embodiments it is preferred that the organic solvent has an E_{T}(30) polarity ≤ 62, such as ≤ 60, ≤ 55 or ≤ 50. E_{T}(30) polarity is measured by the method described in C. Reichardt, Agnew. Chem. Int. Ed., 1979, 18, pages 98-110. Where the solvent comprises a mixture of organic solvents, or is a mixture of organic solvent(s) and water, the E_{T}(30) polarity refers to the solvent as a whole.

In some embodiments it is preferred that the organic solvent has a boiling point of ≤ 120 °C at 1 atm, such as ≤ 100°C or ≤ 90 °C.

The molar equivalents of ligand of Formula (I) added relative to gold is preferably from 1 : 1 to 1 : 4, such as from 1 : 1 to 1 : 2. Including more than 4 equivalents of ligand does not appear to have a detriment to the catalyst performance but is unfavoured on cost grounds. Using 1.2 molar equivalents of ligand is generally sufficient to fully complex the gold.

In step (ii) the support is impregnated with the impregnation solution formed in step (i). Impregnation techniques will be well known to those skilled in the art.

In step (iii) the catalyst is dried to remove the organic solvent. Drying techniques will be well known to those skilled in the art. A typical procedure involves heating the catalyst at a temperature of ≥ 100 °C for a period of 12 hours or more. A stream of gas may be used to help remove the organic solvent. It will be appreciated that the temperature and duration of drying will differ depending on the scale at which the preparation is carried out.

### Catalyst

The invention also relates to catalysts comprising a complex of gold and a ligand of Formula (I) 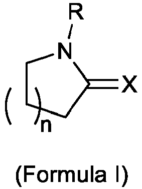 wherein
X is O or S;
n is 1 or 2;
R is H or a C1 to C10 hydrocarbon group optionally including one or more heteroatoms selected from halogen (F, Cl, Br, I), oxygen or nitrogen; and
the complex is supported on a support.

It is preferred that X is O. The use of ligands in which X is S tend to have lower activities compared to when X is O.

In one embodiment R is a C1 to C10 hydrocarbon group including one or more heteroatoms selected from halogen (F, Cl, Br, I), oxygen (e.g. OH) or nitrogen (e.g. NH₂), e.g. a C1 to C6 hydrocarbon group. For example a C1-C6 hydrocarbyl group or a C1-C3 hydrocarbyl group, in each case including one or more heteroatoms selected from halogen (F, Cl, Br, I), oxygen (e.g. OH) or nitrogen (e.g. NH₂). The presence of heteroatoms in group R may be beneficial to improve the solubility of the ligand in a high polarity solvent.

In one embodiment R is a C1 to C10 hydrocarbyl group consisting of C and H atoms only, e.g. a C1 to C6 hydrocarbyl group. For example a C1-C6 hydrocarbyl group or a C1-C3 hydrocarbyl group. The absence of heteroatoms in group R may be beneficial to improve the solubility of the ligand in a low polarity solvent.

It is preferred that R is H or Me, preferably R is Me. The use of ligands in which R is H tend to have lower activities compared to when R is Me.

It is preferred that n is 1.

Preferred ligands are N-methyl-2-pyrrolidone (NMP) and N-methyl-2-piperidone. These ligands show equivalent or better acetylene conversion than a comparative catalyst comprising a gold thiosulphate complex. NMP is a particularly preferred ligand.

For the avoidance of doubt, NMP has the following structure:

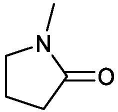

The role of the ligand is to stabilise Au in the Au(I) or Au(III) oxidation state. The method of the present invention makes it possible to produce catalysts having a high dispersion of Au and a high proportion of Au(I) and Au(III) species, which are believed to be the active species for hydrochlorination.

The loading of Au is a trade-off between the cost of Au and the activity of the catalyst. A typical Au content for the catalyst is typically from 0.01 to 5 wt%, based on the weight of catalyst as a whole. Typically the loading of Au will be from 0.01 to 2 wt%, such as from 0.05 to 1.5 wt%, such as from 0.2 to 1.5 wt%.

In some embodiments, in addition to Au the catalyst may include one or more promoter metals. The presence of a promoter metal may improve the activity of the catalyst and/or help to maintain the activity of the catalyst over time. Suitable promoters include Group 1 and Group 2 metals, as well as cobalt, copper, lanthanum and cerium.

Any known catalyst support may be used to make the catalyst of the invention. Typical metal oxide supports such as alumina, silica, zeolite, silica-alumina, titania or zirconia and composites thereof may be used. It is preferred that the support is a carbon support. The carbon may be derived from natural sources (e.g. peat, wood, coal, graphitic) or may be a synthetic carbon. The carbon is preferably an activated carbon, activated for example by steam, acid, or otherwise chemically activated.

The support may be in the form of a powder, granules or shaped particles. Examples of shaped particles include spheres, tablets, cylinders, multi-lobed cylinders (e.g. trilobes), rings, miniliths etc. or a massive catalyst unit such as a monolith. Alternatively, the catalyst in the form of a powder may be included in a coating formulation and coated onto a reactor wall or shaped substrate such as a monolith. One preferred form of catalyst support comprises a plurality of shaped units in the form of cylinders, spheres or lobed cylinders each having a diameter of 0.1 - 10 mm, or, more preferably a diameter in the range 1 - 3mm. In the case of a cross-section shape having a non-uniform diameter, such as a lobed cylinder, the diameter is an average diameter. Cylinders and trilobes are particularly preferred shapes of support.

### Uses of the catalyst

It is envisaged that the catalysts of the present invention will be useful in any chemical process where gold containing catalysts are known to find utility. The catalysts are particularly suitable for hydrochlorination of compounds containing an alkyne moiety, especially for the conversion of acetylene to VCM.

The conversion of acetylene to VCM is typically carried out at elevated temperature, usually between about 100°C and 250°C. The reaction temperature is a balance between conversion and economics of running the reactor at higher temperatures. Furthermore, at temperatures significantly above 200 °C coking can become significant. Surprisingly, catalysts comprising a ligand of Formula (I) are active at lower temperatures than existing Au-based catalysts.

The HCI and acetylene are preferably premixed, and also preferably pre-heated to the reaction temperature. Normally HCI is present in excess of the amount required for the stoichiometric reaction. The catalyst may be present in the reactor in the form of a fixed bed of catalyst particles arranged such that the feed gases are passed over or through the catalyst bed. Alternative reactor arrangements may be used, including fluidised beds or other moving bed arrangements. The catalyst may alternatively be provided in the form of a monolith or coated on the wall of a reactor vessel. The catalyst bed may be provided with means to regulate the temperature to avoid overheating due to the exothermic reaction, or to raise the temperature, if required. It may be preferred to treat the catalyst with HCl before use in the process. This treatment is typically carried out by flowing HCI over the catalyst for a period of at least an hour at a temperature of at least 50°C, more especially > 100°C. This pre-treatment may take place in the reactor by operating with a flow of HCI without the acetylene, at a suitable temperature.

### Examples

### General procedure for catalyst testing

A sample of the catalyst (~ 90 mg) was loaded onto glass wool inside a reactor tube. A feed stream was prepared by combining 24.00 mL/min C₂H₂ (5% acetylene in Argon), 30.00 mL/min HCI (5% HCI in Argon) and 4.10 mL/min Argon. The temperature of the feed stream was set at 180 °C unless otherwise specified. The acetylene conversion was determined by gas chromatography.

### Example 1 - preparation of comparative catalyst without ligand (E1)

Catalyst E1 was prepared via an impregnation method. Activated carbon (NORIT ROX 0.8) was initially ground and sieved (150 mesh) to obtain a powder with a particle size < 150 µm. The gold precursor, HAuCl₄·3H₂O (solid from Alfa Aesar, 20 mg, assay 49%) was dissolved in dry acetone (2.7 mL) and allowed to stir for 10 mins. The solution was added drop-wise, with stirring, to the ground, activated, dry carbon powder (0.99 g). The solution was left to stir for 1 h at room temperature and finally dried under nitrogen at 45 °C for 16 h.

### Example 2 to 11 - role of ligand

Examples 2 to 11 reported in Table 1 were prepared by the same method as described for Example 1, modified as follows. The ligand was weighed into a vial. The solution of HAuCl₄·3H₂O in acetone was added to the ligand and the resulting solution was used for impregnation of the carbon powder as described in Example 1. The ratio of Au : ligand (mol : mol) was 1 : 1.2, 1 : 2 or 1 : 4. The final Au loading was 1 wt%.

**Table 1**

| Catalyst | Ligand | Ligand : Au (mol : mol) | Catalyst | Ligand | Ligand : Au (mol : mol) |
|---|---|---|---|---|---|
| E1 | none | n/a | E7 | 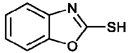 2-mercaptobenzoxazole | 1.2 |
| E2-1.2 | 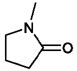 N-methyl pyrrolidone | 1.2 (E2-1.2) | E8 | 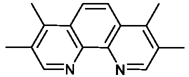 3,4,7,8-tetramethylphenanthroline | 1.2 |
| E2-2.0 | | 2.0 (E2-2.0) | | | |
| E2-4.0 | | 4.0 (E2-4.0) | | | |
| E3 | 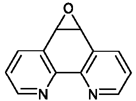 epoxyphenanthroline | 1.2 | E9 | 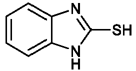 2-mercaptobenzimidazole | 1.2 |
| E4 | 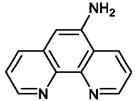 Phenanthroline - amine | 1.2 | E10 | 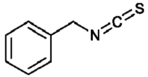 benzylisothiocyanate | 1.2 |
| E5 | 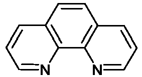 Phenanthroline | 1.2 | E11 | 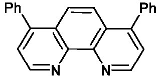 Bathophenanthroline | 1.2 |
| E6 | 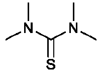 N,N,N,N-Tetramethylthlourea | 1.2 | | | |

Ligands E3-E11 are not according to the present invention.

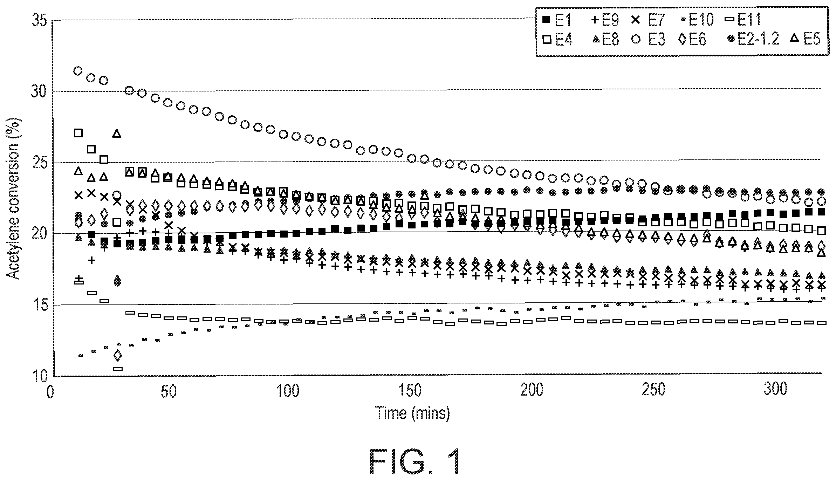

These catalysts were tested for their acetylene conversion following the general procedure. The results are shown in Figure 1. With the exception of E1 (no ligand), E2-1.2 (NMP) and E10 (benzyl isothiocyanate) all of the catalysts deactivated during the course of the test.

### Role of ligand equivalents

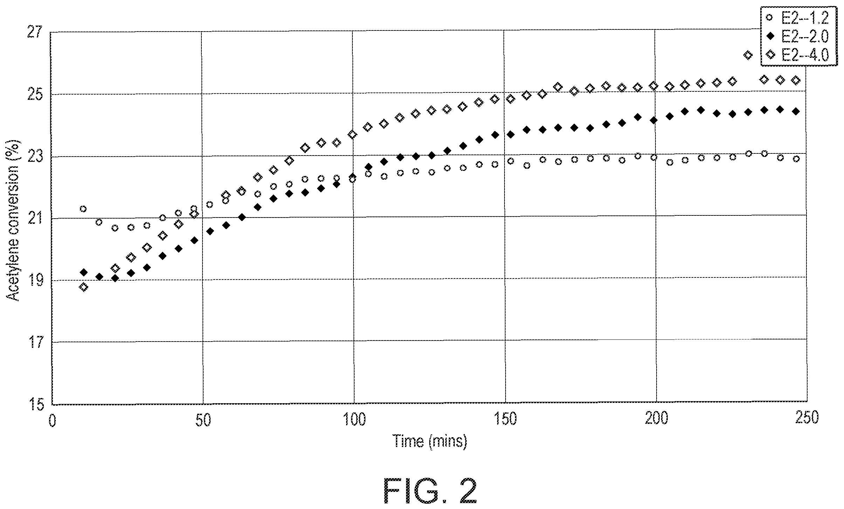

The role of ligand equivalents for E2-1.2, E2-2.0 and E2-4.0 is compared in Figure 2. The performance was similar but in general E2-4.0 > E2-2.0 > E2-1.2.

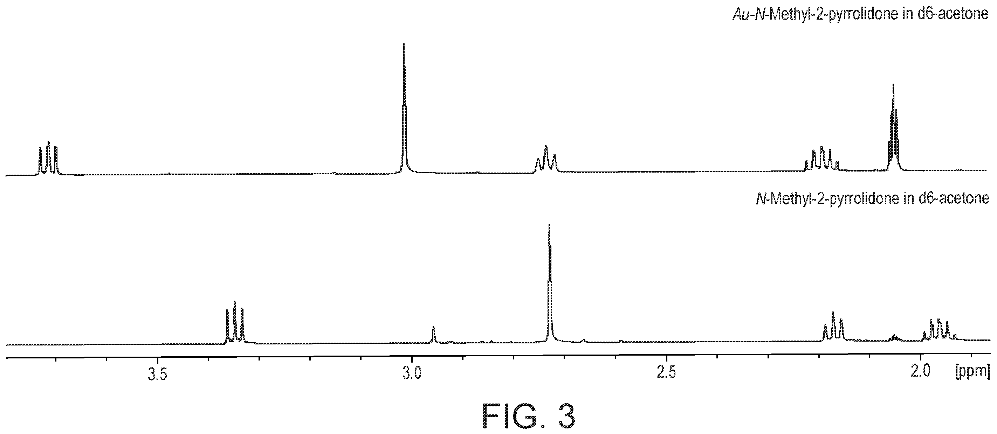

An overlay of the ¹H NMR spectrum of NMP in d₆-acetone and E2-4.0 prepared in d₆-acetone is shown in Figure 3. All of the peaks associated with NMP are shifted in the complex suggesting that the NMP is coordinated.

### Role of temperature

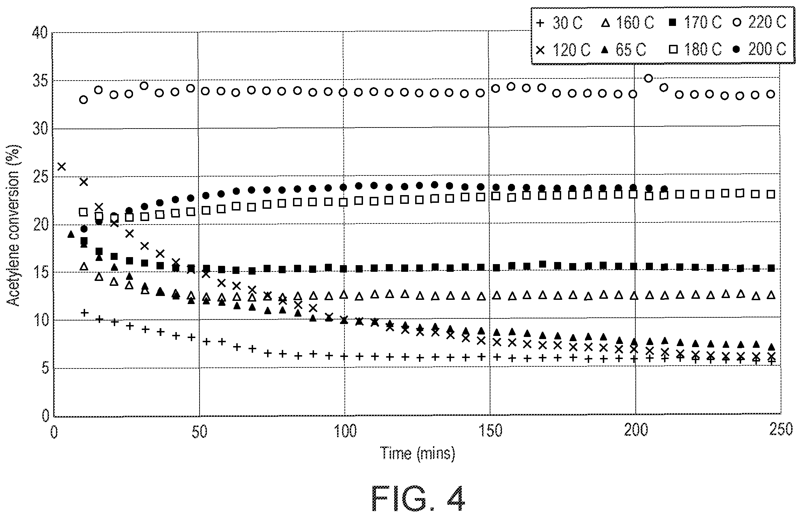

The activity of Example E2-1.2 was tested at temperatures of 30, 65, 120, 180, 200 and 220 °C. The results are shown in Figure 4. Activity increased with increasing temperature. Although the acetylene conversion was highest at 220 °C, the amount of acetylene converted did not correspond to the amount of VCM produced. It is thought that this discrepancy is related to the formation of coke at high temperatures.

### Examples 12 to 18 - alternative ligands

The catalysts in Table 2 were prepared following the same procedure as Examples 2-11 using 1.2 equivalents of the ligand. The final Au loading was 1 wt%.

**Table 2.**

| Catalyst | Ligand | Catalyst | Ligand |
|---|---|---|---|
| E12 | 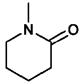 N-methyl-2-piperidone | E16 | 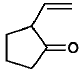 1-vinyl-2-pyrrolidinone |
| E13 | 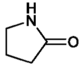 2-pyrrolidinone | E17 | 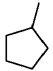 methylcyclopentane |
| E14 | 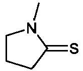 1-methylpyrrolidine-2-thione | E18 | 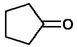 cyclopentanone |
| E15 | 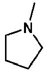 1-methylpyrrolidine | | |

Ligands E15-E18 are not according to the present invention.

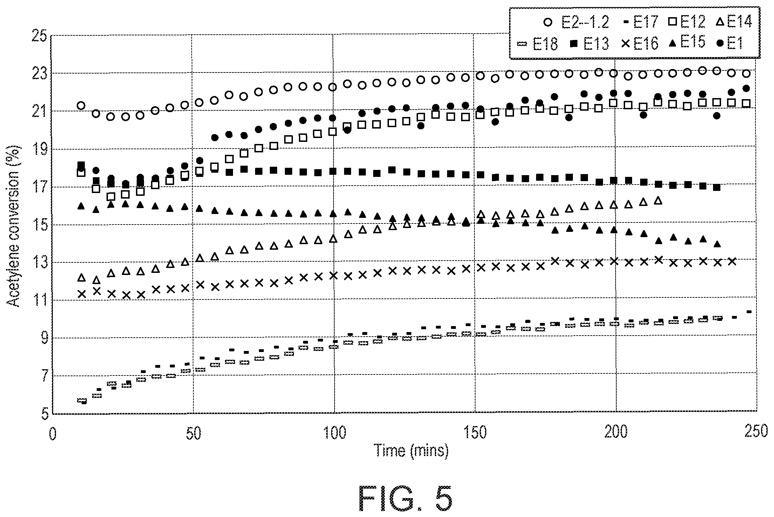

These catalysts were tested for their acetylene conversion following the general procedure. The results are shown in Figure 5. Catalyst E2-1.2 containing the NMP ligand showed the highest activity of all catalysts and had an activity which was relatively stable over time. Catalyst E12 containing the N-methyl-2-piperidone ligand also showed good activity which was relatively stable, but slightly less active than E1 containing thiosulphate ligands.

The other catalysts were less active and, in the case of E13 containing 2-pyrrolidinone and E15 containing 1-methylpyrrolidine, showed gradual deactivation from the start.

### Examples 19 and 20 - Role of solvent (reference examples, not according to the invention)

Catalysts containing 1% Au, in each case as a complex with thiosulphate ligand, were prepared by the following procedure.

Activated carbon (NORIT ROX 0.8) was ground to a powder and sieved to < 180 micron. Ammonium thiosulphate (0.975g) and CaCl₂ (0.175g) were dissolved in 17 mL demineralized water. An HAuCl₄ solution (Johnson Matthey, 0.25g Au, assay 41.76%) was diluted with 17 mL demineralized water and this solution as added to the ammonium thiosulphate containing solution. The carbon powder (24.75 g dry weight) was impregnated with the gold containing solution by an incipient wetness technique. The impregnated mass was allowed to stand for 1h and then dried in air at 105 °C for 16h to obtain catalyst E19.

Activated carbon (NORIT ROX 0.8) ground to a powder and sieved < 180 micron). Ammonium thiosulphate (0.975g) was dissolved in 15 mL demineralized water. An HAuCl₄ solution (Johnson Matthey, 0.25g Au, assay 41.76%) was diluted with 25 mL acetone and this solution as added to the ammonium thiosulphate containing solution. The carbon powder (24.75g dry weight) was impregnated with the gold containing solution by an incipient wetness technique. The impregnated mass was allowed to stand for 12h at room temperature in flowing air and then dried in air at 105 °C for 16h to obtain catalyst E20.

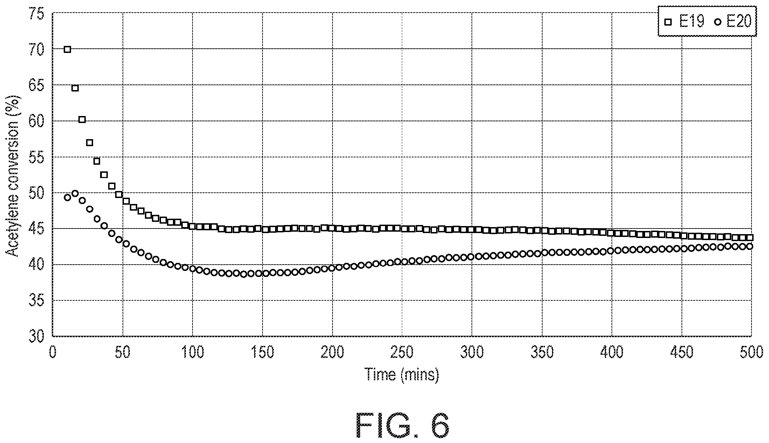

Catalyst testing was carried out using a fritted reactor tube. 150 mg of SiC was placed on the frit followed by 300 mg of the catalyst. A sample of the catalyst (~ 300 mg) was loaded onto glass wool inside a reactor tube. A feed stream was prepared by combining (5.83 mL/min acetylene (5% acetylene in Argon)) and 6.03 mL/min HCI (5% HCI in Argon). The temperature of the feed stream was set at 180 °C unless otherwise specified. The acetylene conversion was determined by gas chromatography. The results are shown in Figure 6.

The catalyst prepared using an aqueous impregnation solution with 1% Au (E19) originally showed ~ 70% conversion and stabilised at ~ 45% conversion. The stable value was ~ 65% of the initial activity.

In contrast, the catalyst prepared using an acetone/water impregnation solution (E20) originally showed ~ 50% conversion and stabilised at ~ 45% conversion. The stable value was ~ 90% of the initial activity. This catalyst showed a much more stable activity profile without high initial activity. This is expected to be advantageous in avoiding local hot-spots on initial activation of the catalyst.

### Examples 21 and 22 - alternative sulfur-containing ligand (reference examples, not according to the invention)

A catalyst containing 1% Au (E21) was prepared following the procedure used to produce E20, except that aqueous solution of ammonium thiosulphate was replaced by a solution of sodium thiocyanate (0.530g) dissolved in 25 mL acetone. The solution of the HAuCl₄ solution was diluted with 15 mL acetone rather than 25 mL. The solutions were mixed together and immediately used to impregnate the carbon powder.

A catalyst containing 0.5% Au (E22) was prepared following the procedure used to produce E21, except that 0.125 g Au as HAuCl₄ solution was used instead of 0.25 g Au as HAuCl₄ solution. aqueous solution of ammonium thiosulphate was replaced by a solution of sodium thiocyanate (0.530g) dissolved in 25 mL acetone. The solution of the HAuCl₄ solution was diluted with 15 mL acetone rather than 25 mL. The solutions were mixed together and immediately used to impregnate the carbon powder.

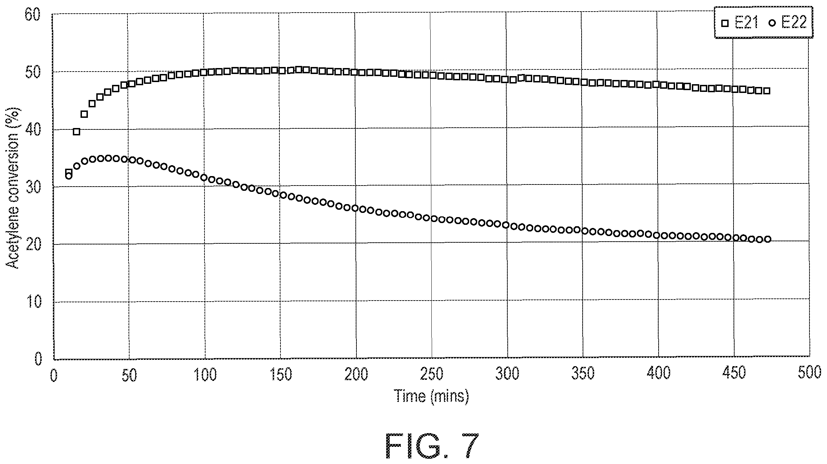

The results are shown in Figure 7. In each case, the catalyst showed a stable activity profile without an initial spike in catalyst activity. This is expected to be advantageous in avoiding local hot-spots during initial activation of the catalyst.

### Preparation of [HAuCl₄](NMP)₂

Acetone (15 ml) was added to 2.50g of a solution of HAuCl₄ (40.02% Au) containing 1.00g Au. To this solution was added a solution containing 4 molar equivalents of N-methyl pyrrolidine (2.01 g) in 15 ml acetone. The resultant solution was mixed and allowed to react for 30 min. Thereafter the solution was evaporated under vacuo to give an oily residue. After washing with aliquots of cyclohexane a solid product was obtained and recrystallised from 1-propanol - cyclohexane to yield a crop of fine bright yellow needles in high yield. Needle shaped single crystals suitable for X-ray crystal structure analysis were obtained by slow crystallisation from 1-propanol - cyclohexane.

Elemental analysis calculated for AuC₁₀Cl₄H₁₉N₂O₂ requires Cl, 26.4%; C, 22.3%; H, 3.6%; N, 5.2%; found Cl, 26.4%; C, 22.3%; H, 3.5%; N, 5.2%.

Single crystal structure determination by X-Ray diffraction analysis at 296 K using radiation with a wavelength of 0.71073 Å showed that the material had the following properties: monoclinic, space group P2₁/n, a = 14.2719(8) Å, b = 7.6086(5) Å, c = 16.5447(15) Å, α = 90 °, β = 98.485(7) °, γ = 90 °, *Z* = 4, crystal dimensions 0.380 x 0.140 x 0.090 mm³. A total of 4383 independent reflections were collected.

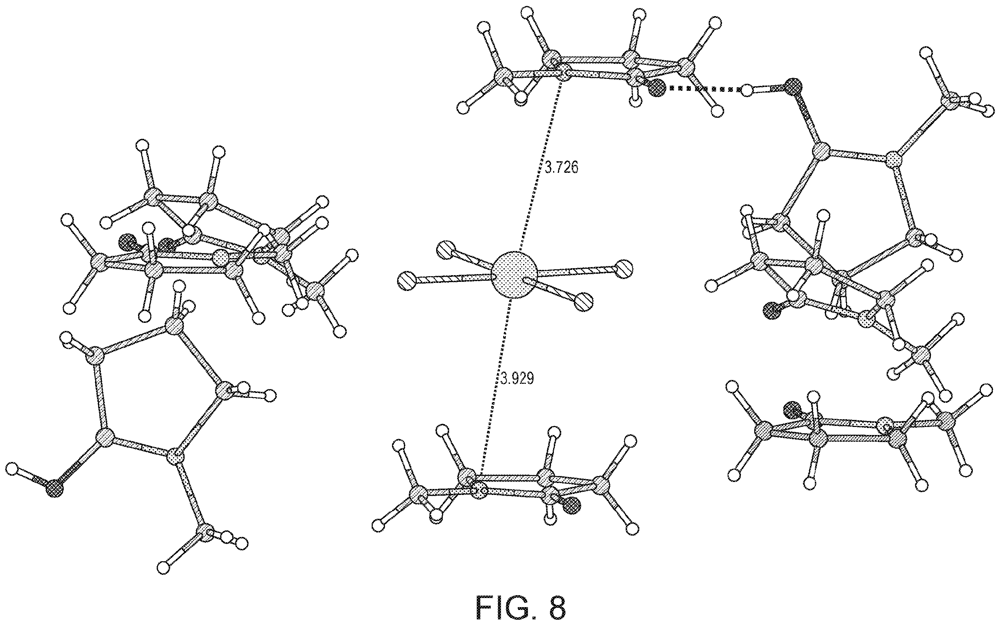

An image of the structure determined, showing NMP coordination around the AuCl₄ centre, is shown in Figure 8. The N-Au distances were 3.726 Å and 3.929 Å.

## Claims

1. A method for the production of a hydrochlorination catalyst, comprising the steps of:
i) preparing an impregnation solution by combining a source of gold and a ligand in a solvent, wherein the solvent comprises an organic solvent which comprises or consists of acetone;
ii) impregnating a support with the impregnation solution from step (i); and
iii) drying the product of step (ii) to obtain the catalyst;
wherein the ligand is of Formula (I) 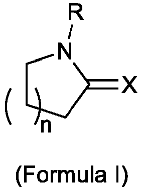
wherein
X is O or S;
n is 1 or 2;
R is H or a C1 to C10 hydrocarbon group optionally including one or more heteroatoms selected from halogen (F, Cl, Br, I), oxygen or nitrogen.

2. A method according to claim 1, wherein the solvent consists of the organic solvent.

3. A method according to claim 1, wherein the solvent consists of a mixture of organic solvent and aqueous solvent.

4. A method according to any of claims 1 to 3, wherein the ligand is N-methyl-2-pyrrolidone.

5. A hydrochlorination catalyst comprising a complex of gold and a ligand of Formula (I) 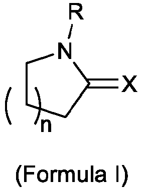 wherein
X is O or S;
n is 1 or 2;
R is H or a C1 to C10 hydrocarbon group optionally including one or more heteroatoms selected from halogen (F, Cl, Br, I), oxygen or nitrogen; and
the complex is supported on a support.

6. A catalyst as claimed in claim 5, wherein the ligand is N-methyl-2-pyrrolidone.

7. A catalyst as claimed in claim 5 or claim 6, wherein the support is in the form of a powder, granules or shaped particles.

8. A catalyst as claimed in any of claims 5 to 7, wherein the support is a carbon support.

9. A process for the catalytic hydrochlorination of a substrate containing an alkyne unit, wherein the reaction is carried out in the presence of a catalyst according to any of claims 5 to 8 or a catalyst producible by a method according to any of claims 1 to 4.

10. A process as claimed in claim 9, wherein the substrate is acetylene.

## Patentansprüche

1. Verfahren zum Herstellen eines Hydrochlorierungskatalysators, umfassend die Schritte:
i) Herstellen einer Imprägnierlösung durch Kombinieren einer Goldquelle und eines Liganden in einem Lösungsmittel, wobei das Lösungsmittel ein organisches Lösungsmittel umfasst, das Aceton umfasst oder daraus besteht;
ii) Imprägnieren eines Trägers mit der Imprägnierlösung aus Schritt (i); und
iii) Trocknen des Produkts aus Schritt (ii), um den Katalysator zu erhalten;
wobei der Ligand die Formel (I) hat 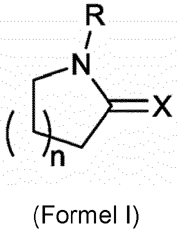
wobei
X O oder S ist;
n 1 oder 2 ist;
R H oder eine C1- bis C10-Kohlenwasserstoffgruppe ist, optional einschließlich eines oder mehrerer Heteroatom(e), ausgewählt aus Halogen (F, Cl, Br, I), Sauerstoff oder Stickstoff.

2. Verfahren nach Anspruch 1, wobei das Lösungsmittel aus dem organischen Lösungsmittel besteht.

3. Verfahren nach Anspruch 1, wobei das Lösungsmittel aus einer Mischung aus dem organischen Lösungsmittel und einem wässrigem Lösungsmittel besteht.

4. Verfahren nach einem der Ansprüche 1 bis 3, wobei der Ligand N-Methyl-2-pyrrolidon ist.

5. Hydrochlorierungskatalysator, umfassend einen Komplex aus Gold und einem Liganden der Formel (I) 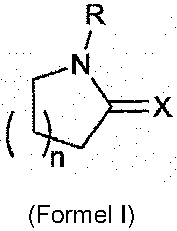
wobei
X O oder S ist;
n 1 oder 2 ist;
R H oder eine C1- bis C10-Kohlenwasserstoffgruppe ist, optional einschließlich eines oder mehrerer Heteroatom(e), ausgewählt aus Halogen (F, Cl, Br, I), Sauerstoff oder Stickstoff; und
der Komplex auf einem Träger ist.

6. Katalysator nach Anspruch 5, wobei der Ligand N-Methyl-2-pyrrolidon ist.

7. Katalysator nach Anspruch 5 oder 6, wobei der Träger in Form eines Pulvers, Granulats oder geformter Partikel ist.

8. Katalysator nach einem der Ansprüche 5 bis 7, wobei der Träger ein Kohlenstoffträger ist.

9. Verfahren zur katalytischen Hydrochlorierung eines Substrats, das eine Alkineinheit enthält, wobei die Reaktion in Gegenwart eines Katalysators nach einem der Ansprüche 5 bis 8 oder eines Katalysators, der durch ein Verfahren nach einem der Ansprüche 1 bis 4 herstellbar ist, ausgeführt wird.

10. Verfahren nach Anspruch 9, wobei das Substrat Acetylen ist.

## Revendications

1. Procédé destiné à la production d'un catalyseur d'hydrochloration, comprenant les étapes consistant à :
i) préparer une solution d'imprégnation en combinant une source d'or et un ligand dans un solvant, dans lequel le solvant comprend un solvant organique qui comprend ou est constitué d'acétone ;
ii) imprégner un support avec la solution d'imprégnation provenant de l'étape (i) ; et
iii) sécher le produit de l'étape (ii) pour obtenir le catalyseur ;
dans lequel le ligand est de Formule (I) 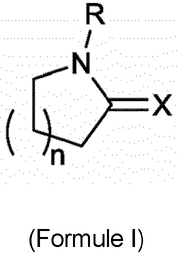
dans lequel
X est O ou S ;
n vaut 1 ou 2 ;
R est H ou un groupe hydrocarboné en C1 à C10 comportant facultativement un ou plusieurs hétéroatomes choisis parmi halogène (F, Cl, Br, I), oxygène ou azote.

2. Procédé selon la revendication 1, dans lequel le solvant est constitué du solvant organique.

3. Procédé selon la revendication 1, dans lequel le solvant est constitué d'un mélange de solvant organique et de solvant aqueux.

4. Procédé selon l'une quelconque des revendications 1 à 3, dans lequel le ligand est N-méthyl-2-pyrrolidone.

5. Catalyseur d'hydrochloration comprenant un complexe d'or et un ligand de Formule (1) 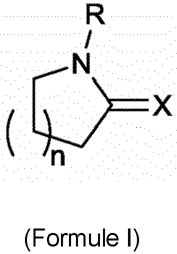
dans lequel
X est O ou S ;
n vaut 1 ou 2 ;
R est H ou un groupe hydrocarboné en C1 à C10 comportant facultativement un ou plusieurs hétéroatomes choisis parmi halogène (F, Cl, Br, I), oxygène ou azote ; et
le complexe est supporté sur un support.

6. Catalyseur selon la revendication 5, dans lequel le ligand est N-méthyl-2-pyrrolidone.

7. Catalyseur selon la revendication 5 ou la revendication 6, dans lequel le support est sous la forme d'une poudre, de granules ou de particules profilées.

8. Catalyseur selon l'une quelconque des revendications 5 à 7, dans lequel le support est un support en carbone.

9. Processus destiné à l'hydrochloration catalytique d'un substrat contenant un motif alcyne, dans lequel la réaction est effectuée en présence d'un catalyseur selon l'une quelconque des revendications 5 à 8 ou d'un catalyseur pouvant être produit par un procédé selon l'une quelconque des revendications 1 à 4.

10. Processus selon la revendication 9, dans lequel le substrat est de l'acétylène.
